# FASCICULE DE BREVET EUROPEEN

(11) **EP 0 689 449 B1**
(45) Date de publication et mention de la délivrance du brevet: **30.10.2002**
(21) Numéro de dépôt: 94910445.9
(22) Date de dépôt: 21.03.1994
(51) Int. Cl.: A61K 38/14

(54) **COMPOSITIONS POUR L'APPLICATION EN THERAPEUTIQUE HUMAINE, CARACTERISEES PAR L'ASSOCIATION D'UN MURAMYL-PEPTIDE A UNE CYTOKINE**
ZUSAMMENSETZUNGEN FÜR DIE ANWENDUNG IN MENSCHLICHER THERAPIE, CHARAKTERISIERT DURCH DIE ASSOZIATION EINES MURAMYLPEPTIDS MIT EINEM CYTOKIN
THERAPEUTICAL COMPOSITIONS FOR USE IN HUMANS, CHARACTERISED BY A COMBINATION OF A MURAMYL PEPTIDE AND A CYTOKINE

(30) Priorité: 19.03.1993 FR 9303230; 31.03.1993 FR 9303787
(43) Date de publication de la demande: 03.01.1996
(73) Titulaire: VACSYN S.A., 75015 Paris (FR)
(72) Inventeur: CHEDID, Louis, F-75015 Paris (FR); BAHR, Georges, F-92800 Puteaux (FR); LEFRANCIER, Pierre, F-91190 Gif-sur-Yvette (FR)
(74) Mandataire: Desaix, Anne
(86) Numéro de dépôt international: FR9400307
(87) Numéro de publication internationale: WO94021275

(56) Documents cités:
- EP-A- 0 228 833
- EP-A- 0 257 890
- EP-A- 0 329 609
- BRITISH JOURNAL OF CANCER vol. 63, no. 3 , 1991 pages 399 - 403 S.T.A. MALIK ET AL 'Therapy of human ovarian cancer xenografts with intraperitoneal liposome encapsulated muramyl-tripeptide phosphoethanolamine ( MTP-PE) and recombinant GM-CSF'
- JOURNAL OF BIOLOGICAL RESPONSE MODIFIERS vol. 9, no. 1 , Février 1990 pages 98 - 102 P.R. WYDE ET AL 'Muramyl peptides and polyinosinic-polycytodylic acid given to mice prior to influenza virus challenge reduces pulmonary disease and mortality'
- CHEMICAL ABSTRACTS, vol. 112, no. 19, 7 Mai 1990, Columbus, Ohio, US; abstract no. 176682u, SANCEAU J. ET AL 'Secretion of interleukin-6 (IL-6) by human monocytes stimulated by muramyl dipeptide and tumor necrosis factor alpha' page 554 ;colonne R ; & IMMUNOLOGY vol. 69, no. 1 , 1990 pages 52 - 56
- ADVANCES IN EXPERIMENTAL MEDICINE AND BIOLOGY vol. 319 , 1992 pages 253 - 263 ICHIRO AZUMA ET AL 'Stimulation of host-defense mechanism with synthetic adjuvants and recombinant cytokines against viral infection in mice'
- JOURNAL OF INTERFERON RESEARCH vol. 11, no. SUP1 , Novembre 1991 page S162 P. POUILLART ET AL 'In vitro potentiation by murametide of interferon antiviral activity against encephalomyocarditis infection'
- JOURNAL OF INTERFERON RESEARCH vol. 11, no. SUP1 , Novembre 1991 page S257 P. POUILLART ET AL 'Comparison of the therapeutic effect of synthetic muramyl peptides derivatives on the antiviral activity of interferon'

## Description

Les interférons (plus particulièrement l'interféron α) sont sécrétés par les leucocytes. Ils sont à ce titre classés parmi les cytokines, médiateurs produits majoritairement par les cellules du système immunitaire. D'une façon générale, les cytokines peuvent être considérées comme des hormones caractéristiques du système immunitaire. La possibilité de produire des cytokines par recombinaison génétique a ouvert la voie à des études plus élaborées des comportements de certaines de ces cytokines, notamment d'interférons recombinants chez l'animal. Parmi les interférons, le plus étudié est l'interféron α (IFN α) qui se subdivise en IFN α 2a, IFN α 2b et IFN 2c.

En particulier, l'activité antivirale de certains interférons de souris, et même la potentialisation de cette activité chez la souris par certains dérivés de la N-acétyl-muramyl-L-alanyl-D-isoglutamine (MDP) ou d'homologues de ceux-ci ont déjà été mises en évidence. En revanche, comme l'indique F. Dianzani dans son article intitulé "Interferon Treatments: how to use an Endogenous System as a Therapeutic Agent" (Traitements par l'interféron : Comment utiliser un système endogène en tant qu'agent thérapeutique) dans le "Journal of Interferon Research, Special Issue, May 1992, Mary Ann Liebert, Inc., Publishers, pp.109-118, les nombreux essais cliniques tentés chez l'homme avec des interférons ont été "plus que décevants". En effet, bien que les essais chez l'animal paraissaient ouvrir des potentialités thérapeutiques étendues, leurs effets secondaires mal contrôlés n'ont autorisé à ce jour leur utilisation que dans un nombre restreint d'indications thérapeutiques. Aussi a-t-il été proposé à ce jour de n'utiliser l'IFN α 2a que dans un nombre réduit de "niches" comme l'indique aussi le "Scrip's Cytokines Report", PJB publications Ltd. 1993, à propos des difficultés rencontrées dans l'application en thérapeutique humaine des interférons :
**a)** dans le traitement des cas d'hépatites B et C les plus graves. Les traitements consistent en administrations quotidiennes ou à raison de trois fois par semaine de 5 à 10 millions d'unités pendant 3 à 6 mois. Des résultats positifs sont observés dans 30 à 40% des cas ;
**b)** dans le domaine du cancer, le traitement de la tricholeucémie chevelue (Hairy Cell Leukemia) et de la leucémie chronique myélogène, les applications envisagées sont le SIDA, le syndrome de Kaposi, le myélome multiple, le mélanome et certains types de carcinome.

La gravité de ces syndromes est telle qu'elle a aussi entrainé l'acceptation des graves effets secondaires accompagnant l'utilisation de l'IFN α en thérapeutique humaine. Quatre vingt dix huit pour cent des patients ainsi traités souffrent en effet d'un syndrome grippal, souvent extrêmement sévère, accompagné de nausées, de vomissements, de troubles du système nerveux central et périphérique et de troubles cardiaques. Ces effets, sont attribués au moins en partie à l'induction par l'interféron de médiateurs pyrétiques qui pourraient être l'IL-1 et des prostaglandines dont la production serait activée par l'interféron. Il s'y ajoute des accès de fatigue, d'anorexie, de perte de poids, qui pourraient être liés au moins en partie à la production de TNF et/ou l'expression accrue de récepteurs de TNF également induits par les interférons. Toute procédure qui optimiserait les effets bénéfiques des interférons ou des cytokines thérapeutiquement utiles, tout en optimisant aussi les productions ou l'activité des cytokines ou d'autres médiateurs biologiques indésirables telles que IL-1, IL-8 et/ou TNF (abréviation de l'expression anglaise "Tumor Necrosis Factor" ou facteur de la nécrose tumorale), ne pourrait être que vouée à un échec certain.

Enfin les administrations d'interférons s'accompagnent souvent de fortes leucopénies et thrombopénies accompagnées d'un blocage de la maturation de précurseurs myéloïdes. Ces phénomènes entraînent la nécessité d'interrompre périodiquement les traitements à base d'interférons, pour chaque fois autoriser une régénération de la formule sanguine.

Autant d'inconvénients qui, jusqu'à ce jour, n'autorisent pas une exploration des possibilités réelles d'utilisation des interférons en thérapeutique humaine, hormis pour le traitement des quelques syndromes rappelés plus haut.

Des difficultés semblables sont rencontrées avec d'autres cytokines dont l'intérêt thérapeutique serait certain s'il était possible de remédier à ces difficultés. Il en serait ainsi par exemple de IL-2, IL-3, IL-6, G-CSF, M-CSF, GM-CSF, etc... de TNF et de l'IL-1, dont les potentialités thérapeutiques ne peuvent être exploitées que très rarement.

On sait qu'il a été suggéré d'utiliser en particulier IL-3 et certains CSF pour accélérer la récupération hématopoïétique chez des patients frappés d'aplasie de moelle (qui se manifeste sous l'effet d'un facteur pathogène endogène et/ou iatrogène), en particulier à l'issue de traitements chimiothérapiques ou chez des patients ayant subi des transplantations de moelle. Mais l'utilisation de IL-3 en clinique humaine ou des CSFs les plus actifs (par exemple GM-CSF) se heurte dans la pratique aux mêmes difficultés que l'interféron α.

A ces inconvénients d'ordre thérapeutique, s'ajoutent les coûts extrêmes des traitements actuels a base de cytokines therapeutiquement utiles. Les doses administrées, pour autant qu'on puisse en réguler l'effet in vivo, sont en effet considérables par rapport aux doses thérapeutiquement utiles, ce qui s'explique peut être par le fait que contrairement aux hormones endocrines, les cytokines n'agissent pas à distance mais seulement sur les cellules proches des cellules sécrétrices. En d'autres termes, toute cytokine administrée n'atteignant pas les cellules cibles peut être considérée comme "thérapeutiquement perdue".

EP-A-228 823, EP-A-329 609, EP-A-257 890, Malik et al, *British Journal of Cancer,* 63(3) pp. 399-403 (1991) et Azuma et al, *Advances in Experimental Medicine and Biology,* 319, pp.253-263 (1992) décrivent des agents immunostimulants comprenant un muramyl-peptide et une cytokine, mais ces muramyl-peptides présentent une structure différente de celle des présents muramyl-peptides.

Sanceau et al, *Chemical Abstracts,* 112 (19) 1990, Abstract No. 17668u décrit l'utilisation d'un muramyl-dipeptide, utilisé sans cytokine, pour la stimulation de la sécrétion de l'interleukine-6.

Pouillart et al, *Journal of Interferon*, Res. 11, Sup 1 p. 162 (Nov. 1991) constate que le muramétide est capable de potentialiser l'activité antivirale de l'interféron dans une culture de cellules.

Pouillart et al, *Journal of Interferon*, Res. 11, Sup 1 p. 257 (Nov. 1991) fait référence à des essais chez la souris et met en oeuvre des analogues de MDP et des interférons α/β de souris et mentionne qu'il existe une corrélation entre la conformation de différents analogues de MDP et leur capacité à potentialiser les actions des interférons.

Wyde et al, *Journal of Biological Response Modifiers*, 9 (1) pp. 98-102 (1990) décrit une combinaison de muramyl-dipeptide et de poly-IC active contre le virus de l'influenza chez la souris. Wyde et al décrivent aussi une combinaison de muramyl-dipeptide et d'interféron-alpha de souris. Ils constatent que ladite combinaison n'est pas active contre le virus de l'influenza. Ils exposent en outre que le remplacement dans cette composition du muramyl dipeptide par le muradimétide ne l'a pas davantage rendu active contre le virus de l'influenza.

L'invention a pour but de remédier au moins en grande partie à ces inconvénients et difficultés.

La possibilité de bénéficier des effets thérapeutiques de l'IFN α ou de cytokines thérapeutiquement utiles autres que l'IFN α, le cas échéant en doses plus réduites, sans provoquer d'effets secondaires insupportables serait un pas très important tant sur le plan thérapeutique que sur le plan économique. C'est un des objectifs que l'invention s'est fixé.

Plus précisément, l'invention a pour but d'accroître l'efficacité des thérapeutiques mettant en oeuvre un interféron ou, plus généralement, une cytokine ayant une valeur thérapeutique et, même, d'élargir les champs thérapeutiques de ces cytokines (dont naturellement l'interféron), tout en remédiant en grande partie aux inconvénients indiqués ci-dessus, plus particulièrement en rapport avec les interférons.

L'invention repose en grande partie sur la découverte que l'association de certains muramyl-peptides à une cytokine susceptible de présenter un effet thérapeutique chez l'homme rendait désormais possible l'utilisation de certaines de ces cytokines en clinique humaine à des doses efficaces, ce qui ne pouvait auparavant être envisagé sérieusement, et dans le meilleur des cas (par exemple cas de l'interferon) ouvrant la voie à des gammes de traitement thérapeutique pour des indications cliniques en nombre et en effacité considérablement plus importants. Il a en effet été constaté que ces muramyl-peptides permettaient également d'induire la synthèse in vivo d'autres cytokines qui n'apparaissent pas, tout au moins à des taux décelables, à la suite de l'administration seulement de l'un des deux éléments, interféron et muramyl-peptide, d'où par conséquent, un élargissement des spectres d'activité thérapeutique des cytokines et des muramyl-peptides. Parmi les cytokines induites, on mentionnera plus particulièrement, l'interleukine 6 (IL-6), de G-CSF et, d'un antagoniste (IL-1 RA) du récepteur de l'interleukine I. Qui plus est, les administrations associées de certains muramyl-peptides et de l'interféron α (IFN α) permettent la production des effets recherchés en utilisant des doses sub-optimales de cytokine, notamment d'IFN α, grâce à la potentialisation des effets bénéfiques de la cytokine, mais en l'absence d'induction des cytokines indésirables, notamment de IL-1, de TNF ou d'IL-8.

L'invention concerne donc plus particulièrement une association d'au moins une cytokine avec au moins un muramyl-peptide choisi parmi ceux qui, lorsqu'ils sont administrés in vivo en association avec un interféron, induisent également la production accrue in vivo d'un antagoniste IL-1 RA du récepteur de l'interleukine I et, de préférence, n'induisent pas un accroissement des cytokines IL-1, TNF et IL-8.

De préférence, on utilise plus particulièrement ceux des muramyl-peptides qui, répondant aux conditions mentionnées ci-dessus, induisent également, lorsqu'ils sont administrés avec un interféron, une synthèse accrue in vivo de IL-6 ou de G-CSF, ou de préférence les deux à la fois.

L'invention n'est pas limitée à l'association d'un interféron (α, β ou γ) à un tel muramyl-peptide. Les interférons peuvent également être remplacés par d'autres cytokines particulièrement par celles ci-dessus identifiées à titre d'exemple. En d'autres termes, l'invention découle de la capacité de certains muramyl-peptides, lorsqu'ils sont administrés en association avec une cytokine :
- à potentialiser l'activité biologique de la cytokine considérée permettant aussi d'obtenir les effets recherchés après administrations de doses plus faibles que celles considérées actuellement comme thérapeutiquement nécessaires.
- à ne pas favoriser et éventuellement même inhiber l'induction in vivo par cette cytokine des facteurs limitants ses possibilités d'applications thérapeutiques comme il a été observé dans le cas de l'association avec l'IFN et, le cas échéant et simultanément ;
- à induire la production in situ, d'une part, d'inhibiteurs de ces facteurs limitants, par exemple IL-1 RA et, notamment lorsque la cytokine consiste en un interféron, le récepteur soluble du TNF (STNF-R) et, d'autre part, la sécrétion in situ par les cellules compétentes du système lymphoïdes de cytokines, par exemple IL-6 et G-CSF dans le cas des interférons, qui permettent alors l'élargissement du spectre d'action de la cytokine de l'association administrée.

L'invention concerne donc toute association mettant en oeuvre une ou plusieurs cytokines et un muramyl-peptide sélectionné en raison de sa capacité à ne pas induire et même à inhiber chez l'homme la sécrétion par ses cellules compétentes des médiateurs biologiques tels IL-1 et/ou IL-8 et/ou TNF, ou à tout le moins à inhiber leurs effets. On connaît en effet le rôle hautement vraisemblable de ces médiateurs dans les effets secondaires liés à l'administration de certaines cytokines. La capacité du muramyl-peptide à potentialiser l'action de la cytokine tout en protégeant l'hôte contre l'induction des cytokines ou d'autres médiateurs nuisibles peut, dans chaque cas, être vérifiée par des essais comparatifs in vivo, de préférence chez l'homme, faisant intervenir, d'une part, la cytokine choisie seule et, d'autre part, une association de cette cytokine avec le muramyl-peptide choisi, et la détection et la mesure sur une base comparative (par exemple dans des dosages RIA ou ELISA utilisant les anticorps appropriés) des taux des cytokines induites étudiées. Les résultats susceptibles d'être obtenus sont illustrés de façon bien entendu non limitatives dans les exemples qui suivent appliqués à l'interféron α exposés plus loin.

De préférence, les cytokines utilisées seront toujours des cytokines d'origine humaine. Il convient de souligner que cette expression doit être entendue comme couvrant, outre les cytokines naturelles d'origine humaine (on ne peut alors méconnaître les difficultés qu'implique leur isolement en quantités adéquates), les cytokines recombinantes correspondantes produites par des techniques de génie génétique, c'est-à-dire de cytokines néanmoins caractérisées par des séquences en acides aminés identiques à celles de ces cytokines naturelles, ce qui n'exclut néanmoins pas de séquences en acides aminés équivalents différant des précédentes, notamment par des substitutions, addition ou délétions d'acides aminés n'entraînant ni des modifications substantielles des propriétés caractéristiques des cytokines recombinantes à "séquences identiques", ni l'apparition de propriétés nuisibles (par exemple inductions d'anticorps chez l'hôte humain).

De préférence les muramyl-peptides mis en oeuvre dans l'invention sont des muramyl-peptides, notamment choisis parmi ceux qui sont caractérisés par la formule générale suivante : 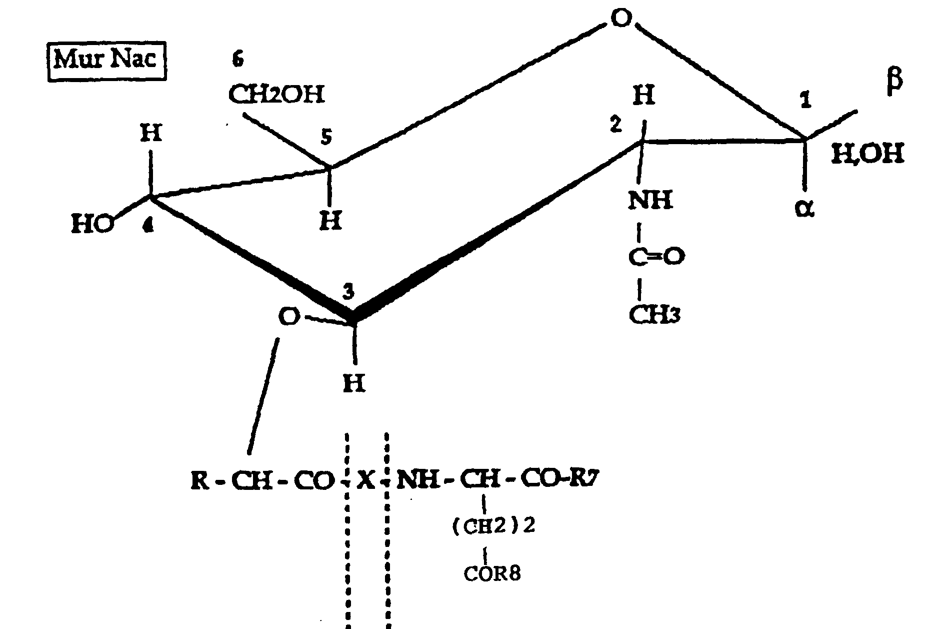 dans laquelle le groupe R est un hydrogène ou un groupe méthyle ; X est L-alanyle, L-leucyle, L-isoleucyle, L-valyle, L-thréonyle, L-(N-méthyl)- alanyle et R₇ et R₈ sont indépendamment l'un de l'autre, des groupes hydroxyle, amino, O(CH₂)ₓH avec x = 1, 2, 3, 4 ou 5 pu des groupes peptidiques comprenant de 1 à 3 résidus d'acides aminés, de préférence levogyres.

Une catégorie de muramyl-peptides préférés sont ceux dans lesquels :
- R = CH₃,
- X est L-alanyle ou L-thréonyle,
- R₇ est un groupe O(CH₂)_{x'} H, avec x' = 1, 2, 3 ou 4,
- R₈ est un groupe amino ou un groupe O(CH₂)_{x"}H, avec x" = 1, 2, 3 ou 4.

Des muramyl-peptides particulièrement préférés sont constitués par le muramétide (R = CH₃, X = L-Ala, R₇ = OCH₃ et R₈ = NH₂, le murabutide (R = CH₃, X = L-Ala, R₇ = OnC₄H₉ et R₈ = NH₂) et enfin le muradimétide (R = CH₃, X = L-Ala, R₇ = R₈ = OCH₃), et le cas échéant leurs homologues dans lesquels le résidu L-alanyle de leur groupe peptidique est remplacé par un groupe thréonyle.

Il va de soi que les précédentes classes de muramyl-peptides à caractère hydrophile ne sont nullement limitatives. Des muramyl-peptides lipophiles, tels que ceux décrits dans le brevet français n°8407340, peuvent également être mis en oeuvre dans le cadre de l'invention.

Il va enfin de soi que l'on peut aussi avoir recours à tous autres muramyl-peptides adjuvants porteurs de substituants en les positions 1, 4 ou 6 du groupe saccharidique, dès lors qu'ils auraient les mêmes effets favorables que les muramyl-peptides préférés mentionnés plus haut.

On remarquera enfin que l'expression "association" n'implique pas que la cytokine et le muramyl-peptide choisis soient nécessairement administrés à l'hôte humain en mélange ou de façons simultanées. Elle s'étend également à toute utilisation ou présentation impliquant leurs administrations à des intervalles de temps non nuls, étant néanmoins entendu que ces intervalles doivent être suffisamment courts pour autoriser les interactions mutuelles des deux constituants de l'association dans les conditions sus-indiquées.

Ce type d'association mettant en oeuvre interleukines et muramyl-peptides est efficace quand les interleukines sont administrées aux doses suivantes:
- pour l'interféron :
   0,05 MU/kg/jour à 10 MU/kg/jour, et de préférence 0,5 MU/kg/jour à 5 MU/kg/jour, une dose optimale étant de 1 MU/kg/jour à 5 MU/kg/jour.
   A titre d'indication, l'activité spécifique de l'IFN utilisé est de 4 à 8 UI/mg de protéines.
- pour l'IL-2 :
   0,3 MU/kg/jour à 10 MU/kg/jour, de préférence entre 1 MU/kg/jour et 2 MU/kg/jour, sachant que l'activité spécifique de l'IL-2, notamment commercialisée par la société Cetus sous le nom de Proleukin, est de l'ordre de 10 millions d'UI par mg de protéines.
- pour le GM-CSF, les doses préférées sont de 1 à 25 µg/kg et par jour et mieux encore de 5 à 10 µg/kg et par jour.
- les muramyl-peptides, notamment le Murabutide, étant administrés à des doses de l'ordre de 10 à 350 µg/kg/j et de préférence 50 à 200 µg/kg/j ; dose de 100 µg/kg/j est dans tous les cas associée à des doses sub-optimales ou optimales de cytokine.

D'autres caractéristiques de l'invention apparaîtront encore à la faveur des exemples non limitatifs décrits ci-après.

Au cours d'essais cliniques effectués chez des volontaires humains sains, de l'IFN α 2a (interféron Roféron A dosé à 3 MU/ml commercialisé par Roche sous la marque ROFERON A, a été administré en association avec des muramyl-peptides, en particulier le muramétide et le murabutide. Il a été montré :
**a)** que la sécrétion de Néoptérine qui est le marqueur biologique reconnu signant l'activité immunostimulante de l'IFN était très fortement augmentée, suite à des administrations de doses sub-optimales d'IFN, associées à des doses de Murabutide ou de Muramétide ; il en est de même de l'antagoniste du récepteur de l'IL-1 (IL-1 RA) dont l'IFN seul induit faiblement la synthèse qui est augmentée considérablement par l'addition de muramyl-peptides montrant ainsi une activité synergique entre IFN et les immunomodulateurs synthétiques ; ceci est également vrai pour le récepteur soluble du TNF (STNF-R) ;
**b)** que les sujets recevant l'association montrent une augmentation significative du nombre de cellules de la lignée blanche par rapport au groupe traité par IFN ;
**c)** qu'il apparaît, dans le plasma des sujets traités par l'association, des médiateurs de l'immunité : Interleukine 6, G-CSF, alors que l'on n'observe l'induction d'aucun des médiateurs pyrogènes et inflammatoires principaux, plus particulièrement des cytokines IL-1, IL-8 et TNF.

Ces constatations reposent plus particulièrement sur les résultats des essais qui suivent.
- **PREMIERE SERIE D'ESSAIS : injection sous-cutanée de mélanges d'interféron α-IFN-α-2a avec soit le Murabutide, soit le Muramétide :**

Des sujets sains subissent une série de tests afin de démontrer leur capacite à être inclus dans l'essai. Ils reçoivent par la voie sous-cutanée les mélanges décrits dans le tableau I suivant de Murabutide (MUB) ou Muramétide (MUM) avec de l'Interféron α 2a (IFN α).

**Tableau I**

| IFN-α A MUB B MUM | 10⁵ U | 3 x 10⁵ U | 10⁶ U | 3 x 10⁶ U | 6 x 10⁶ | O |
|---|---|---|---|---|---|---|
| A 35 µg/Kg | A I | A II | A III | A IV | A V | Témoin A35 |
| A 100 µg/Kg | A VI | A VII | A VIII | A IX | A X | Témoin A100 |
| B 35 µg/Kg | B I | B II | B III | B IV | B V | Témoin B35 |
| B 100 µg/Kg | B VI | B VII | B VIII | B IX | B X | Témoin B100 |
| O | Témoin IFN 10⁵ | Témoin IFN 3 x 10⁵ | Témoin IFN 10⁶ | Témoin IFN 3 x 10⁶ | Témoin IFN 6 x 10⁶ | Placebo |

Les sujets sont suivis pendant les trois jours suivant l'administration de l'IFN et/ou des muramyl-peptides et l'ensemble des tests et des examens nécessaires à la réalisation d'un essai de phase I sont effectués.

### En particulier les examens suivants sont pratiqués :

Analyse hématologique avec comptes de globules blancs au moment de l'injection puis aux temps 6h, 12h, 24h, 36h, 60h et 72 heures après injection.

Des échantillons de sang sont prélevés avant l'injection et 6h, 24h et 48 heure après l'injection, pour recueillir le sérum afin d'effectuer les dosages de Néoptérine, Interleukine 1,6 et 8 G-CSF, IL-1 RA et de TNF-α.

Les comptes leucocytaires sont effectués dans une compteur automatique de cellules COULTER COUNTER.

Les échantillons de sang pour les dosages dans le sérum sont centrifugés à 4°C à 3000 rpm pendant 10' juste après le prélèvement. Les sérums sont stockés à -20°C. Les comptes leucocytaires et les dosages de Néoptérine et de cytokine sont effectués en utilisant des kits commerciaux.

### RESULTATS DES EXAMENS ET DES TESTS

### 1) Effets secondaires :

Les effets secondaires qui ont été répertoriés au cours des essais (tels que migraine, fièvre et douleurs articulaires) ont été très modérés. En particulier, les effets de type syndrome grippal observés aux fortes doses d'IFN 3M et 6M d'unités ne sont pas augmentés par la combinaison avec les muramyl-peptides bien qu'il y ait une forte augmentation de l'activité biologique de l'IFN et apparition d'un profil modifié de cette activité avec synthèse de nouveaux médiateurs biologiques.

### 2) Comptes leucocytaires :

Les neutrophiles sont les cellules qui sont les plus affectées par des traitements leucopéniants. C'est la baisse du taux de neutrophiles qui est responsable de la baisse des défenses immunitaires non spécifiques chez les sujets soumis à la radiothérapie ou à une chimiothérapie cytotoxique et/ou myélotoxique. Pour simplifier la lecture, seuls donc les comptes de neutrophiles ont été donnés à l'heure où leur variation est la plus notable.

**Tableau II**

| Augmentation du nombre de neutrophiles chez les volontaires sains après administration de l'association muramyl-peptides-IFN α. | | | | |
|---|---|---|---|---|
| IFN-α administré | Muramyl peptide administré | | No. | Compte des neutrophiles |
| (Nombre d'unités) | Composé | Dose (µg/Kg) | testé | % ligne de base^{a} |
| 0 | Murabutide | 100 | 6 | 268 ± 43^{b} |
| 0 | Murametide | 100 | 4 | 279 ± 67 |
| 1 x 10⁵ | - | 0 | 6 | 143 ± 34 |
| 1 x 10⁵ | Murabutide | 100 | 6 | 265 ± 61 |
| 3 x 10⁵ | - | 0 | 6 | 169 ± 47 |
| 3 x 10⁵ | Murabutide | 100 | 6 | 292 ± 112 |
| 1 x 10⁶ | - | 0 | 6 | 147 ± 42 |
| 1 x 10⁶ | Murabutide | 100 | 6 | 207 ± 46 |
| 1 x 10⁶ | Murametide | 100 | 6 | 232 ± 78 |
| 3 x 10⁶ | - | 0 | 6 | 144 ± 19 |
| 3 x 10⁶ | Murabutide | 100 | 6 | 252 ± 59 |
| 3 x 10⁶ | Murametide | 100 | 6 | 255 ± 96 |
| 6 x 10⁶ | - | 0 | 6 | 191 ± 75 |
| 6 x 10⁶ | Murabutide | 100 | 6 | 226 ± 74 |
| 6 x 10⁶ | Murabutide | 100 | 6 | 298 ± 71 |

| | | | | |
|---|---|---|---|---|
| **a:** Représentent les valeurs les plus élevées observées pendant 24 heures après l'administration. **b:** moyenne ± déviation standard. | | | | |

### 3) Dosages de la Néoptérine :

Les dosages sont effectués sur les sérums prélevés au temps 0, 24h et 48 heures. Un kit RIA (Radio Immuno Assay) (Behring Diagnostic, France) est utilisé. Les résultats sont exprimés en nanomoles/ml, les valeurs normales varient de 4 à 9 nanomoles/ml.

**Tableau III**

| | | | IFN α | | | | |
|---|---|---|---|---|---|---|---|
| Muramyl peptide | T | 0 | 10⁵ u | 3 x 10⁵ u | 10⁶ u | 3 x 10⁶ u | 6 x 10⁶ u |
| 0 | | Placebo | Témoin IFN | Témoin IFN | Témoin IFN | Témoin IFN | Témoin IFN |
| | 0 | 6.00 ± 0.80 | 6.03 ± 1.58 | 5.78 ± 2.03 | 3.26 ± 0.85 | 3.22 ± 0,73 | 4.87 ± 2.50 |
| | 24 | 5.50 ± 0.40 | 9.02 ± 2.75 | 10.21 ± 2.20 | 9.20 ± 2.13 | 14.39 ± 2.58 | 20.95 ± 3.73 |
| | 48 | 5.20 ± 0.40 | 9.43 ± 2.11 | 11.20 ± 1.37 | 9.72 ±2.90 | 14.80 ± 2.22 | 18.73 ± 2.62 |
| MUB 35 µg/Kg | | | | A II | A III | A IV | A V |
| | 0 | NT | NT | 5,23 ± 1.06 | 5.06 ± 0.77 | 4.26 ± 0.34 | 5.21 ± 0.73 |
| | 24 | | | 9.78 ± 1.18 | 13.00 ± 3.62 | 17.31 ± 2.03 | 22.61 ± 3.94 |
| | 48 | | | 10.67 ± 3.41 | 12.41 ± 2.90 | 18.01 ± 3.56 | 21.30 ± 6.25 |
| MUB 100 µg/Kg | | Témoin MUB | A VI | A VII | A VIII | A IX | A X |
| | 0 | 6.37 ± 0.99 | 5.97 ± 1.78 | 4,91 ± 0.96 | 3.83 ± 0,82 | 4.14 ± 0.49 | 5.85 ± 0.68 |
| | 24 | 7.33 ± 0.75 | 11.30 ±3.64 | 12.43 ± 2.67 | 20,11 ± 6,73 | 19.45 ± 4.09 | 29,14 ± 0,97 |
| | 48 | 8.22 ± 1.39 | 11.76 ± 4.87 | 13.42 ± 2.75 | 18.13 ± 6.92 | 19.18 ± 4.00 | 28.29 ± 0.92 |
| MUM 35 µg/Kg | | | | | B III | B IV | B V |
| | 0 | NT | NT | NT | 4.42 ± 0.70 | 5.67 ± 0.70 | 4.61 ± 1.20 |
| | 24 | | | | 12.48 ± 1.89 | 16.56 ± 1.89 | 21,17 ± 2.17 |
| | 48 | | | | 12.24 ± 2.41 | 14.36 ± 3.47 | 18.33 ± 2.33 |
| MUM 100 µg/Kg | | Témoin MUM | | | B VI | B VII | B VIII |
| | 0 | 5.43 ± 0.66 | NT | NT | 5.20 ± 1.95 | 6.59 ± 1.62 | 4.06 ± 0.17 |
| | 24 | 7.14 ± 1.00 | | | 16.33 ± 2.13 | 20.78 ± 2.90 | 18.41 ± 2.60 |
| | 48 | 7.39 ± 1.37 | | | 14.49 ± 4,72 | 20.33 ± 4.18 | 16.10 ± 2.74 |

On voit sur ce tableau qu'au temps 0 tous les sujets ont des taux normaux de Néoptérine, que les sujets n'ayant reçu qu'un placebo ont un taux constant de Néoptérine et que le Muramétide, ou le Murabutide à la dose de 100 µg/Kg n'influencent pas le taux de façon significative.

Aux doses les plus faibles d'Interféron, l'association avec les muramyl-peptides permet d'obtenir des augmentations significatives du taux de Néoptérine. A la dose la plus forte, le Murabutide administré à 100 µg/Kg permet certes aussi d'obtenir une augmentation significative, mais beaucoup plus faible que celle observée avec l'association.

### 4) Dosages de l'antagoniste du récepteur de l'IL-1 (IL-1 RA) :

Les dosages effectués sur les sérums des sujets ayant reçus 10⁶ unités d'IFN a sont rapportés dans le tableau suivant (tableau IV) et on voit un effet très fort de l'association muramyl-peptide et Interféron (ici aussi kit British Biotechnology Products Ltd.).

**Tableau IV**

| Quantité d'antagoniste du récepteur de l'IL-1 (IL-1 RA) présente dans le sérum de volontaires sains, 6 heures après l'administration de muramyl-peptides et d'IFN α. | | | |
|---|---|---|---|
| IFN-α administré (Nombre d'unités) | Muramyl peptide administré | | Taux sériques d'IL-1 RA (Pg/ml)⁺ |
| | Composé | dose (µg/Kg) | |
| 0 | Murabutide | 100 | 496 ± 326φ |
| 3 x 10⁵ | - | 0 | 258 ± 113 |
| 3 x 10⁵ | Murabutide | 100 | 10 294 ± 16 554* |
| 1 x 10⁶ | - | 0 | 2 093 ± 1 278 |
| 1 x 10⁶ | Murametide | 100 | 16 093 ± 1 621* |
| 1 x 10⁶ | Murametide | 100 | 16 204 ± 1 879* |
| 3 x 10⁶ | - | 0 | 7 332 ± 3 215 |
| 3 x 10⁶ | Murabutide | 100 | 30 563 ± 23 410* |
| 6 x 10⁶ | - | 0 | 15 523 ± 6 554 |
| 6 x 10⁶ | Murabutide | 100 | 66 870 ± 36 554* |

| | | | |
|---|---|---|---|
| +: détectés 6 heures après l'administration de composés, les taux avant administration étaient tous inférieurs aux 200Pg/ml ; | | | |
| φ: moyenne ± déviation standard chez les 6 volontaires d'un même groupe ; | | | |
| *: taux substantiellement différents des taux induits par le seul IFN α (p<0,05-p<0,005 dans le test de Mann Whitney Rank) | | | |

### 5) Dosages de l'IL-6 :

Le dosage est effectué par un kit de dosage Elisa (British Biotechnology Products Ltd.) sur les sérums prélevés au temps 0 et après 6 heures. Les chiffres expriment des pg/ml, les valeurs normales sont inférieures à 6 pg.

**Tableau V**

| IFN-α administré (Nombre d'unités) | Muramyl peptide administré | | Taux sériques d'IL-6 (Pg/ml)⁺ |
|---|---|---|---|
| | Composé | dose (µg/Kg) | |
| 0 | Murabutide | 100 | 2.80 ± 1.60^{S} |
| 0 | Murametide | 100 | 4.30 ± 1.80 |
| 1 x 10⁶ | - | 0 | 2.00 ± 1.70 |
| 1 x 10⁶ | Murabutide | 100 | 15.00 ± 12.00* |
| 1 x 10⁶ | Murametide | 100 | 30.00 ± 30.00* |
| 3 x 10⁶ | - | 0 | 3.60 ± 0.40 |
| 3 x 10⁶ | Murabutide | 100 | 30.00 ± 23.00* |
| 3 x 10⁶ | Murametide | 100 | 31.00 ± 19.00* |
| 6 x 10⁶ | - | 0 | 34.00 ± 56.00 |
| 6 x 10⁶ | Murabutide | 100 | 76.00 ± 58.00 |
| 6 x 10⁶ | Murametide | 100 | 107.00 ± 72.00* |

| | | | |
|---|---|---|---|
| φ: chaque groupe comprenait 6 volontaires sauf celui pour le muramétide qui n'en comprenait que 4 ; +: taux mesurés 6 heures après l'administration ; aucun taux n'a été détecté avant l'administration du composé détecté ; | | | |
| S: moyenne ± déviation standard ; | | | |
| *: taux substantiellement différents des taux induits par le seul IFN α (p<0,05-p<0,005 dans le test de Mann Whitney Rank) | | | |

On voit sur le tableau V qu'à toutes les doses étudiées le Murabutide et l'Interféron ont agi de façon synergique.

### 6) Dosages du G-CSF :

Ces dosages ont été effectués en utilisant le kit de British Biotechnology Products Ltd. (cf. **tableau VI**)

**Tableau VI**

| Quantité de G-CSF présente dans le serum de volontaires sains avant et 6 heures après l'administration de l'association muramyl-peptides et IFN α. | | | | |
|---|---|---|---|---|
| IFN-α administré (Nombre d'unités) | Muramyl peptide administré | | Taux sériques de G-CSF après | |
| | Composé | dose (µg/Kg) | 0 heure | 6 heures |
| 0 | Murabutide | 100 | < 10 | 18 ± 19 |
| 0 | Murametide | 100 | 61 ± 103^{x} | 73 ± 115 |
| 1 x 10⁶ | - | 0 | < 10 | 13 ± 10 |
| 1 x 10⁶ | Murabutide | 100 | < 10 | 58 ± 51^{x} |
| 1 x 10⁶ | Murametide | 100 | < 10 | 98 ± 81^{x} |
| 3 x 10⁶ | - | 0 | < 10 | 17 ± 21 |
| 3 x 10⁶ | Murabutide | 100 | < 10 | 77 ± 65 |
| 3 x 10⁶ | Murametide | 100 | < 10 | 79 ± 83 |
| 6 x 10⁶ | - | 0 | < 10 | 17 ± 26 |
| 6 x 10⁶ | Muabutide | 100 | < 10 | 140 ± 168 |
| 6 x 10⁶ | Murametide | 100 | 12 ± 15 | 284 ± 425^{x} |

| | | | | |
|---|---|---|---|---|
| *: chaque groupe comprenait 6 volontaires, sauf celui qui a reçu le muramétide est le seul qui ne comportait que 4 volontaires ; X: moyenne ± déviation standard ; *: taux substantiellement différents des taux induits par le seul IFN α (p<0,05-p<0,005 dans le test de Mann Whitney Rank) | | | | |

Dans ce cas également on constate une activité synergique des muramyl-peptides et de l'IFN pour induire du G-CSF.

### 7) Dosages du récepteur soluble du TNF (STNF-R) :

Ces dosages sont effectués par un test ELISA dans le sérum des sujets ayant un 6M d'unités d'IFN. A cette dose, il y a déjà une augmentation notable du taux de STNF-R mais l'addition de murabutide permet une augmentation significative de la sécrétion de ce médiateur.

**Tableau VII**

| TRAITEMENT | | Taux de sTNF-R dans le sérum (pg/ml) | | Augmentation nette (pg/ml) |
|---|---|---|---|---|
| IFN - α unités | Murabutide (µg/Kg) | | | |
| | | 0 heure | 6 heures | |
| 0 | 100 | 195 ± 28 | 216 ± 65 | 31 ± 37 |
| 6 x 10⁶ | 0 | 229 ± 37 | 389 ± 95 | 159 ± 108 |
| 6 x 10⁶ | 100 | 232 ± 35 | 576 ± 139 | 345 ± 120* |

| | | | | |
|---|---|---|---|---|
| *: significativement différent des taux induits par l'IFN-α seul (p = 0,005 Mann Whitney Rank Test) | | | | |

### 8) Dosages de l'IL-1, IL-8, THF α :

Ces dosages ont été effectués dans les mêmes conditions et aucune de ces cytokines n'a pu être mise en évidence dans les sérums testés. Les kits utilisés sont tous de British Biotechnology Products Ltd.

### 9) DEUXIEME SERIE D'ESSAIS :

### 9.1. Association d'IFN et de MURABUTIDE :

### 1) Administration unique chez l'homme :

Les sujets ayant participé à l'étude ont été examinés pour déterminer l'influence des différents traitements sur l'apparition éventuelle des signes cliniques suivants : mal de tête, arthralgie-myalgie, fièvre, frissons, asthénie, nausées-vomissements. Le résumé des observations effectuées est donné sur le tableau VIII.

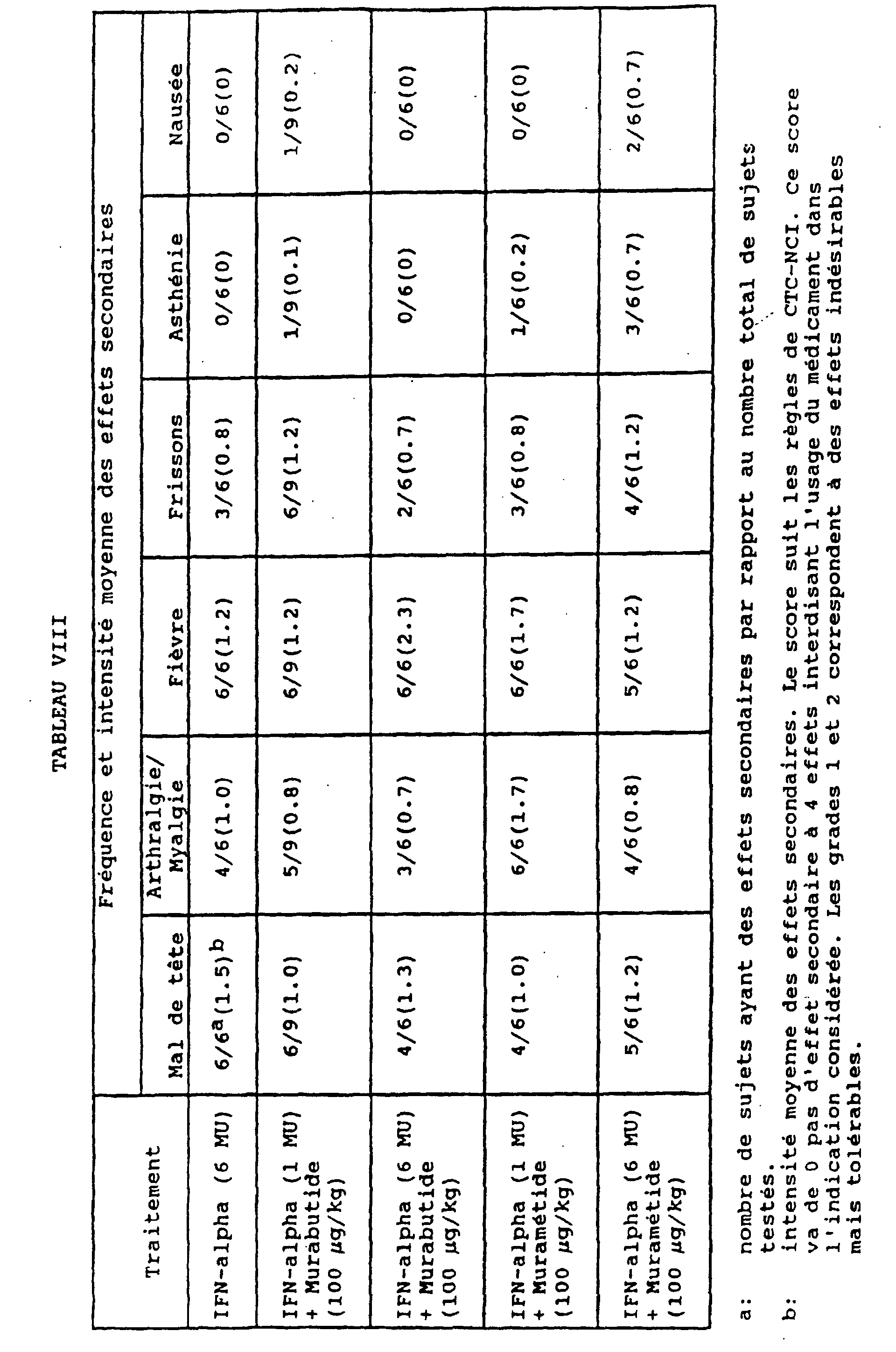

On voit sur ce tableau que les effets secondaires observés sont à peu près équivalents dans tous les groupes. Le groupe qui a reçu les 6 MU d'IFN seul présentant un taux de fréquence et d'intensité moyennes des effets secondaires égal ou supérieur à tous les groupes associant IFN-α et Murabutide ; pour certains de ces effets, on peut observer notamment dans l'association IFN(6 MU) + Murabutide une diminution de la fréquence et de l'intensité des effets secondaires.

Le tableau IX montre les effets biologiques obtenus dans les mêmes groupes, ces résultats indiquant que les effets biologiques obtenus associant le Murabutide ou le Muramétide avec l'interféron α à 1 MU sont au moins égaux ou supérieurs à ceux obtenus avec l'interféron a seul à la dose de 6 MU.

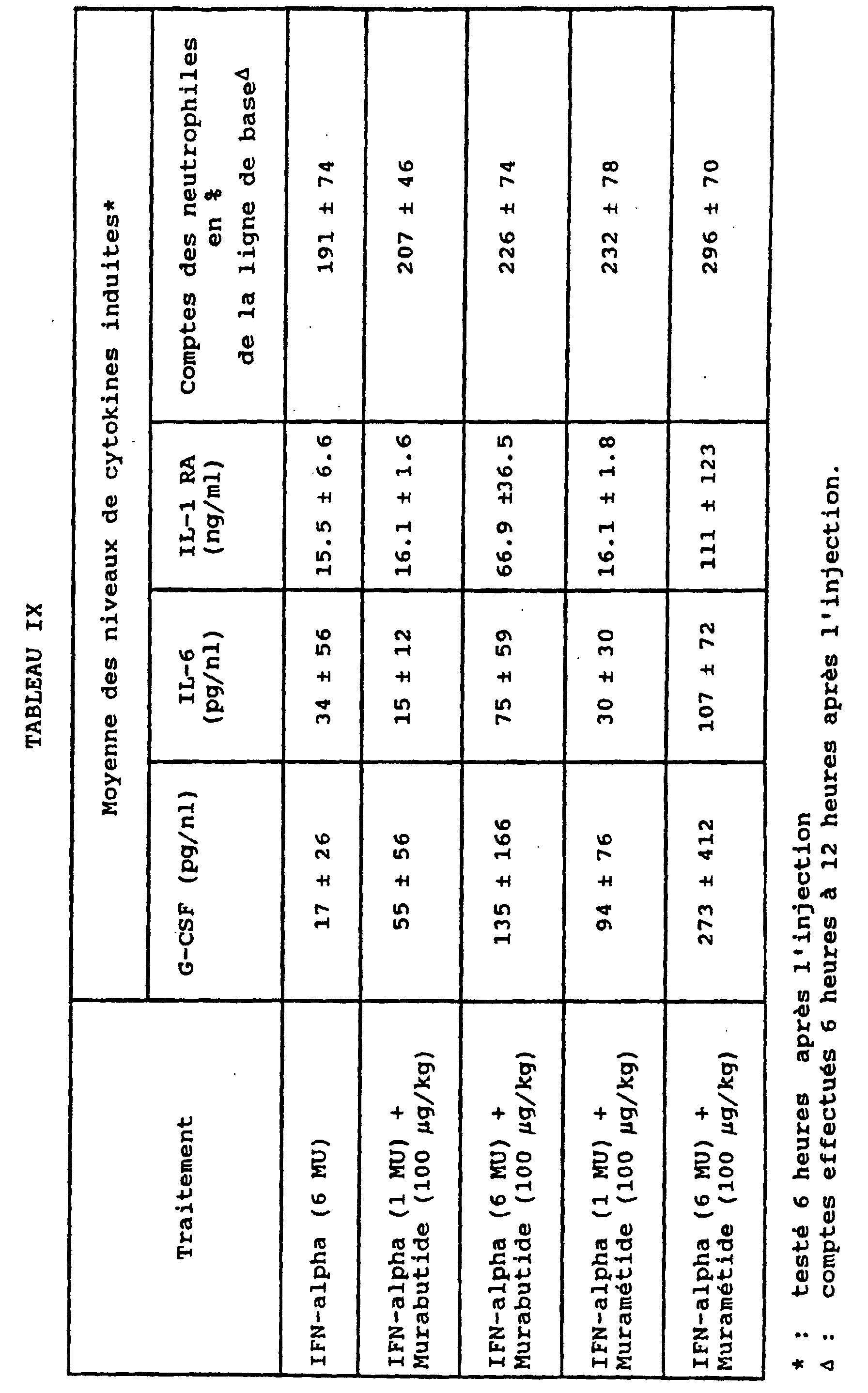

Il est donc très clair d'après ces tableaux que l'invention permet de bénéficier des effets synergiques de l'association Murabutide-cytokines dans les effets recherchés sans que les effets secondaires soient augmentés. Il faut noter que l'administration de 1 MU d'IFN associé au Murabutide ou au Muramétide est plus active que celle de 6 MU d'IFN seul. De plus, l'activité des 6 MU est magnifiée par l'addition de Murabutide ou de Muramétide mais les effets secondaires diminués.

### 2) Administrations répétées chez l'homme :

Des essais de phase I/IIa ont été réalisés sur 3 groupes de 6 volontaires sains qui ont reçu les traitements suivants aux jours 1, 3 et 5 :
a) IFN-α2a(1 MU) Roferon,
b) Murabutide 7 mg
c) l'association des deux immunostimulants.

Ces traitements ont été très bien tolérés et en particulier aucun effet secondaire notable n'a été constaté chez les sujets du groupe C même après la troisième injection.

Le potentiel thérapeutide des traitements a été évalué :
a) en examinant les effets secondaires :
En étudiant leur influence sur le nombre de neutrophiles circulants. Les résultats transcrits sur le tableau X montrent que l'association Murabutide + IFN permet d'obtenir une augmentation du nombre de neutrophiles significativement plus importante que celle obtenue par les immunostimulants administrés seuls et de façon intéressante ce phénomène est aussi marqué après la cinquième qu'après la troisième injection.

**TABLEAU X**

| Traitement | Jour du Traitement | Comptes de neutrophiles (% de la ligne de base) après | | |
|---|---|---|---|---|
| | | 3 heures | 6 heures | 12 heures |
| . IFN-alpha | 1 | 124±27 | 122±30 | 151±20 |
| . Murabutide | 1 | 139±26 | 256±51 | 227±46 |
| . IFN-alpha + murabutide | 1 | 112±22 | 187±70 | 156±60 |
| | | | | |
| . IFN-alpha | 3 | 121±35 | 126±54 | 151±51 |
| . Murabutide | 3 | 133±24 | 182±27 | 176±59 |
| . IFN-alpha + murabutide | 3 | 116±19 | 266±112 | 205±47 |
| | | | | |
| . IFN-alpha | 5 | 104 ± 11 | 117 ± 27 | 149 ± 21 |
| . Murabutide | 5 | 107 ± 19 | 150 ± 28 | 153 ± 23 |
| . IFN-alpha + murabutide | 5 | 106 ± 11 | 210 ± 103 | 205 ± 56 |

b) en analysant l'influence des différents traitements sur l'induction de gènes choisis pour leur importance dans la manifestation de l'activité biologique de l'interféron. Ces expériences ex vivo ont été effectuées sur les cellules du sang circulant des sujets traités.

Les produits des gènes suivants : PKR, MxA, 2-5 OAS, 9-27 et 15 Kd sont les médiateurs des activités antitumorales et antivirales de l'IFN, il est donc important de vérifier que le Murabutide associé à l'IFN permet d'obtenir des niveaux d'induction au moins égales à ceux observés avec l'IFN seul.

L'analyse des mRNAs produits par les cellules des sujets des différents groupes a été effectuée par la technique des "Northern blots" et par hybridation avec des sondes nucléotidigues spécifiques suivant Schreck et al 1992, Clin. Exp. Immunol. vol. 90, p. 188-192. Cette étude a porté sur des cellules prélevées 4 heures après les traitements car c'est le temps le plus précoce pour observer l'influence de l'IFN sur l'induction des gènes sensibles à son action.

Les résultats obtenus montrent que l'association Murabutide-IFN est au moins aussi active que l'IFN seul pour activer les gènes considérés.

L'ensemble des résultats obtenus dans les essais cliniques où l'association Murabutide-IFN a été administrée 3 fois à deux jours d'intervalle montre :
- que des traitements répétés sont aussi bien tolérés que les administrations uniques,
- que l'activité biologique de l'association se manifeste après la troisième administration avec la même amplitude qu'après la première en particulier en ce qui concerne l'augmentation du nombre des neutrophiles et l'induction des gènes impliqués dans les activités antivirales et antitumorales.

### 3) Expériences in vitro sur l'influence de l'association Murabutide et IFN-α sur la synthèse de médiateurs par les monocytes humains :

Du sang prélevé à des volontaires sains a été incubé in vitro avec différentes combinaisons associant le Murabutide et l'IFN-α. Le Murabutide a été utilisé à la dose de 20 µg/ml et l'IFN de 30 à 100 µ/ml. A différents temps au cours de cette incubation, des échantillons du sang sont prélevés et les cellules mononucléées séparés sur gradient de Ficoll. Le RNA total est extrait, purifié et séparé par électrophorèse. Il est ensuite analysé suivant la méthode de Schreck afin de déterminer par technique d'hybridation quels sont les gènes qui ont été induits par le traitement. Cette technique a permis de montrer que des cytokines très importantes sont produites par les cellules sous l'influence de l'association Murabutide-IFN-α à côté des cytokines comme l'IL-1 RA, le sTNFR2, l'IL-6 et le G-CSF dont la présence peut être décelée dans le sérum. Les résultats indiqués dans le tableau XI et obtenus in vitro confirment les observations faites au cours des essais (décrits plus haut) effectués in vivo chez des volontaires sains et montrant la présence d'IL-1 RA, de sTNFR2, d'IL-6 et de G-CSF dans les sérums.

D'autres cytokines, l'IFN-γ et l'IL-12, dont le rôle de médiateurs intercellulaires est très important, ne peuvent être décelées dans la circulation mais les techniques d'hybridation ont montré des quantités élevées de leur RNAs correspondants. Ces cytokines sont très impliquées dans la défense contre les infections intracellulaires.

**TABLEAU XI**

| Incubation des cellules | | Taux moyens des cytokines sécrétées | | |
|---|---|---|---|---|
| IFN-alpha | Murabutide 20 µg/ml | IL-6 pg/ml | sTNFR2 pg/ml | IL-1 RA ng/ml |
| - | - | 1.1±1.6 | 958±290 | 0.3±0.2 |
| - | + | 15.5±11.3 | 1171±478 | 7.8±6.0 |
| 30 u | - | 1.9±1.7 | 1098±222 | 3.5±4.6 |
| 30 u | + | 460.8±330.9 | 1953±483 | 50.8±43.8 |
| 100 u | - | 1.5±0.6 | 1150±286 | 5.0±4.5 |
| 100 u | + | 400.8±313.4 | 1486±625 | 52.0±55.2 |

### 4) Expériences in vivo chez la souris swiss montrant l'activité de l'association du Murabutide-IFN dans un modèle de choc toxique:

Ces expériences montrent l'effet de l'association du Murabutide-IFN dans un modèle de choc toxique et, en particulier, un effet protecteur contre le choc endotoxique et une activité synergique avec l'interféron.

L'administration de D-galactosamine avec du lipopolysaccharide (LPS) dans des souris swiss a pour propriété d'induire un choc endotoxique avec au moins 70% de mortalité dans les 24-48 heures. L'effet prophylactique ou thérapeutique du Murabutide a été étudié dans ce modèle afin d'évaluer son activité anti-inflammatoire lorsqu'il est administré seul ou en combinaison avec IFN-α/β. Le prétraitement des souris avec le Murabutide ou avec l'interféron α/β ne fait pas apparaître un effet prophylactique à l'égard d'un challenge avec le mélange galactosamine/LPS (Tableau XII). Au contraire, le prétraitement avec une combinaison de Murabutide et d'interféron α/β induit une protection significative avec une incidence de mortalité de seulement 33% (contre 78% dans les contrôles).

L'administration de Murabutide dans les souris 1 heure après l'induction du choc endotoxique a un effet curatif significatif qui est bien meilleur que celui induit par l'administration de l'interféron α/β. D'autre part, le traitement avec une combinaison de Murabutide et d'interféron α/β démontre une activité thérapeutique hautement synergique avec près de 80% de protection des souris contre la mortalité due au choc endotoxique.

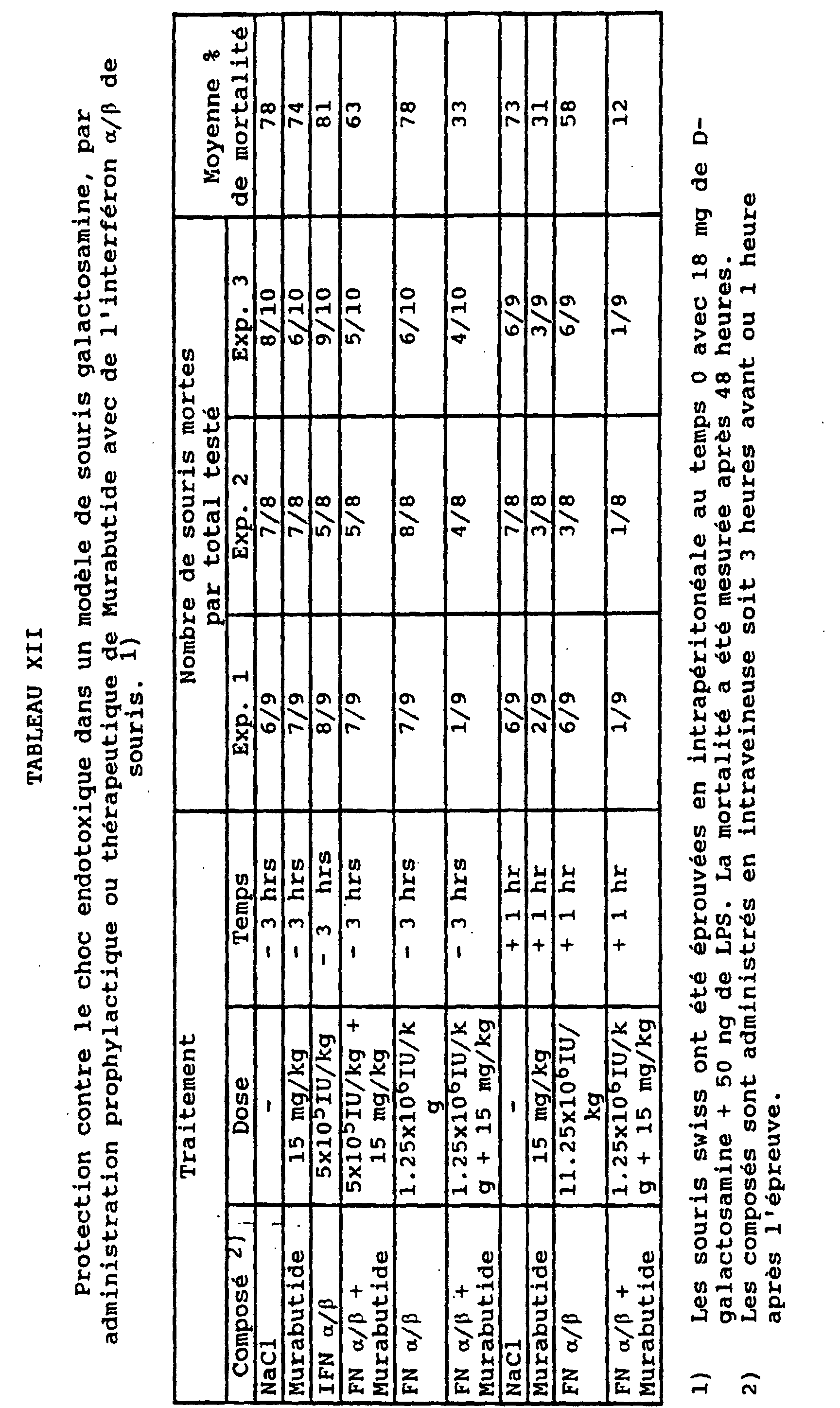

Les résultats montrent clairement que la combinaison d'interféron α/β + Murabutide conduit à une diminution drastique de la mortalité des souris éprouvées par la D-galactosamine et le LPS, que ce soit en prophylactique (33% de mortalité contre 75% à 81% en l'asbence de cette composition) et 12% de mortalité en thérapeutique contre 30% à 73% en l'absence de la combinaison IFN-α/β + Murabutide.

### 9.2. Association avec le GM-CSF :

Des études similaires à celles décrites plus haut ont été réalisées pour étudier l'association Murabutide-GM-CSF. Ces études ont été faites in vitro sur les cellules de volontaires sains après incubation avec les immunomodulateurs seuls ou associés.
a) Analyse au niveau de RNA :
Chez tous les donneurs testés, on a pu observer une induction de la synthèse du récepteur de l'interféron-γ et de la synthèse de l'IL-1RA.
b) Analyse des surnageants :
Certaines cytokines ont été recherchées et mesurées dans les surnageants (voir tableau XIII). Cette analyse confirme que des quantités importantes d'IL-1 RA sont produites sous l'influence de l'association indiquant que le Murabutide devrait permettre une meilleure tolérance du GM-CSF dont une partie des effets secondaires sont liés à des phénomènes inflammatoires.
Les résultats obtenus dans ces expériences indiquent donc que comme dans le cas de l'IFN, l'association Murabutide-GM-CSF permet l'induction importante de médiateurs n'apparaissant pas lors des traitements séparés. De plus, l'induction de ces médiateurs indique que le Murabutide doit augmenter la tolérance à l'administration de GM-CSF.

**TABLEAU XIII**

| Incubation des cellules | | Taux moyens des cytokines sécrétées | | |
|---|---|---|---|---|
| Cytokine (ng/ml) | Murabutide 20 µg/ml | IL-6 pg/ml | sTNFR2 pg/ml | IL-1 RA ng/ml |
| - | - | 1.1±1.6 | 958±290 | 0.3±0.2 |
| - | + | 15.5±11.3 | 1171±478 | 7.8±6.0 |
| GM-CSF(1) | - | 2.0±1.5 | 1027±624 | 4.0±1.4 |
| GM-CSF(1) | + | 34.8±25.2 | 1396±341 | 57.1±26.9 |
| GM-CSF(5) | - | 2.3±2.1 | 1359±350 | 8.7±10.0 |
| GM-CSF(5) | + | 52.8±70.9 | 1970±545 | 76.3±47.0 |

### 9.3. Association avec l'IL-2 :

### 1) Expérience in vivo chez la souris utilisant le Muramétide :

L'IL-2 a montré avoir des effets thérapeutiques importants dans des cas de carcinomes rénaux métastasiques et de mélanomes malins. Son utilisation est néanmoins limitée par ses effets toxiques très importants qui produisent un syndrome ressemblant au choc toxique qui peut être léthal dans 25% des cas.

Un modèle murin a été développé chez la souris C3H/HeN. Des cellules de sarcome KHT sont administrées par la voie veineuse. Après 4 jours et 6 jours elles sont traitées par :
a) le muramétide seul,
b) l'IL-2,
c) l'association des deux immunomodulateurs,
d) un quatrième groupe n'est pas traité.
   - Muramétide en combinaison avec l'interleukine-2 comme traitement en immunothérapie pour les cancers disséminés :

L'immunothérapie avec l'IL-2 a été démontrée comme efficace dans le traitement des carcinomes métastatiques de cellules rénales et de mélanomes malins. En dépit de son efficacité, l'administration d'IL-2 est accompagnée d'une série d'effets secondaires de type septique qui ont limité les doses administrables et restreint son utilisation uniquement dans des centres médicaux spécialisés. Quand il est administré avec l'IL-2, le peptide adjuvant du muramétide réduit la toxicité de l'IL-2 chez les souris porteuses de tumeurs. Les souris recevant l'IL-2 seule s'agglutinent entre elles et sont généralement inactives. Dans les études de survie, les souris recevant la combinaison d'IL-2 et du muramétide survivent à celles recevant l'IL-2 seule. Bien que le muramétide seul n'a aucune activité antitumeur dans la souris porteuse de métastases de sarcome pulmonaire, la combinaison d'IL-2 et de muramétide est plus efficace que l'IL-2 seule. Ces données préliminaires suggèrent que le muramétide peut être utile en combinaison avec l'IL-2 comme moyen de réduire la toxicité de fortes doses de traitement à l'IL-2.

### 2) Expériences in vitro sur des cellules humaines utilisant le Murabutide :

Dans des expériences réalisées sur des cellules de volontaires sains, il a été montré que l'association IL-2 avec le Murabutide permet d'obtenir un taux très élevé de RNA codant pour l'IFN-γ et l'IL-12. Ces 2 cytokines sont essentielles pour l'activité antitumorale. Il faut noter qu'il n'y a pas de synthèse de TNF dont la production lors de traitements par IL-2 est responsable d'une grande partie des effets secondaires en particulier la cachexie.

L'ensemble des résultats in vivo et in vitro obtenus indique clairement que l'association du Murabutide avec l'IL-2 doit permettre un meilleur effet thérapeutique avec moins d'effets secondaires à la fois parce que des doses d'IL-2 moins importantes seront nécessaires et parce que le Muramédite ou le Murabutide diminuent les effets toxiques de l'IL-2.

Les résultats obtenus montrent donc ce qui suit :
**A)** Notamment à propos de l'interféron:
   **a)** A des doses pour lesquelles il est incapable de modifier significativement le taux de synthèse de Néoptérine lorsqu'il est administré seul, l'association de l'IFN α avec le Murabutide ou le Muramétide permet d'obtenir des taux importants de ce marqueur. Aux doses plus élevées d'IFN, l'association permet une élévation significative de son taux. La néoptérine étant considérée comme un des marqueurs principaux de l'activité de l'interféron au niveau des macrophages humains, ces résultats montrent donc que les effets de l'IFN α liés à l'activation de ces cellules peuvent être obtenus avec des doses réduites, bien tolérées ; une remarque similaire s'applique à l'IL-1 RA dont on observe des taux extrêmement élevés après administration de l'association. On peut même observer qu'il y a une activité synergique entre IFN et murabutide ou muramétide et que l'on obtient un effet-dosé, puisqu'approximativement un doublement du taux d'IL-1 RA est obtenu quand la dose d'IFN est doublée. La même observation peut être faite pour STNF-R qui est induit par IFN seul mais faiblement en comparaison des taux induits par l'association.
   **b)** L'association l'IFN avec le muramyl-peptide permet d'obtenir des comptes leucocytaires plus élevés que ceux des témoins ;
   **c)** L'association des muramyl-peptides avec l'IFN α a de plus provoqué la sécrétion sélective d'autres cytokines ou médiateurs immunitaires. En effet, on peut noter chez les sujets traités par l'association, qu'il y a des différences significatives entre leur taux sérique d'IL-6, de G-CSF et de STNF-R et ceux des sujets traités par l'IFN seul ou le Murabutide seul. Par contre, on n'observe pas d'augmentation des taux d'IL-1, d'IL-8 ou de TNF α. Cet effet sélectif, de l'association des muramyl-peptides avec l'Interféron sur la sécrétion de cytokines est tout à fait inattendu et particulièrement intéressant. Il ne pouvait être prédit à partir des propriétés connues des deux composants administrés seuls. Cette découverte offre des possibilités d'applications très intéressantes.
B) Le G-CSF est la cytokine responsable de la différentiation des cellules souches vers la lignée granulocytaire et de leur régénération après un traitement myélotoxique et l'IL-6 et est également très impliquée dans l'hématopoïèse. Ainsi la possibilité d'associer à l'activité de l'IFN α exogène les activités des G-CSF et IL-6 endogènes induites par cet IFN a exogène administré à l'hôte, constitue une avance thérapeutique importante. En effet :
   a) cette association se fait dans des conditions d'effets secondaires minimums ; les doses d'IFN nécessaires sont peu élevées ;
   b) elle intervient dans des conditions beaucoup plus physiologiques de dosages et de disponibilité que si l'on administrait un "cocktail" des cytokines correspondantes ;
   c) l'absence de sécrétion de TNF, d'IL-1 et d'IL-8 est un élément particulièrement favorable, d'autant plus qu'il y a sécrétion de STNF-R qui se combinant à d'éventuelles quantités de TNF neutraliserait son action comme le ferait IL-1 RA dans l'éventualité d'un quantité active d'IL-1. Cet ensemble de données conduit à de nouvelles applications thérapeutiques en médecine humaine et à un élargissement des domaines d'application thérapeutique existants. En particulier :
      **1.** toutes les situations où l'intérêt thérapeutique de l'IFN α peut désormais être mis en évidence. En particulier, l'invention permet l'extension des interférons sous cette forme d'associations à des traitements d'affections tumorales qui ne sont guère envisagées à ce jour, essentiellement en raison de la trop grande importance relative des effets secondaires examinés plus haut. On peut penser au sarcome de Kaposi à la leucémie chronique myéloide, à différents carcinomes, au myélome multiple, au mélanome, à diverses leucémies et lymphomes. L'invention permet, grâce à l'effet de stimulation exercée quant à la production de cytokines thérapeutiquement utiles, de reconstituer au moins en partie les systèmes complexes biologiques physiologiques normalement impliqués dans le maintien de l'homéostase, grâce à l'interaction de leurs constituants avec d'autres facteurs de régulation solubles ou associés aux cellules.
      **2.** plus particulièrement les situations impliquant un déficit des lignées granulocytaire spontané, comme dans le cas de syndromes myélodisplasiques, ou induit par des traitements médicamenteux, notamment à base de cytokines. L'un des grands avantages de l'utilisation de l'association conforme à l'invention réside dans la protection de l'hôte contre les actions leucopéniantes des mêmes cytokines utilisées seules, de sorte que des composés cytotoxiques mettant en oeuvre la cytokine appropriée, notamment pour le traitement de cancers, de maladies infectieuses, ou de déficiences d'origine génétique peuvent être prolongés jusqu'à autoriser un effet curatif plus sûr. En d'autres termes, l'association permet soit un ralentissement de la déplétion, soit même une suffisante restauration du système granulocytaire (voire même une réparation de la moelle) chaque fois que celle-ci est affectée par un traitement cytotoxique (chimiothérapie ou radiothérapie cancéreuse, traitement du SIDA par l'AZT) et/ou immunosuppresseur. Une association peut aussi être utilisée dans des situations conformes à l'invention (cytokine et muramyl-peptide) où les agents cytotoxiques utilisés sont différents de la cytokine de l'association, concurremment avec le traitement cytotoxique du genre en question, ou chaque fois que le traitement avec l'agent cytotoxique est interrompu aux fins de permettre une restauration des phagocytes, des plaquettes sanguines et une stimulation de la moelle osseuse. Enfin, l'invention ouvre la voie à une reconstitution plus rapide du système hématopoiétique chez des personnes dont le système immunitaire avait été auparavant inhibé, voire même détruit, par exemple pour autoriser une transplantation de moelle osseuse ou plus généralement de tissus ou organes allogènes.
         L'induction d'IL-6 permet aussi de penser que l'association à un effet sur la mégacharyocytopoïèse et donc sur la régénération des plaquettes. Ceci a d'ailleurs été observé chez quelques uns des sujets qui ont été suivis sur deux semaines.
         Dans le cadre de cet effet antineutropénique ou antileucopénique, l'invention est également relative à des compositions comportant une association d'une cytokine, par exemple l'interféron ou une interleukine en association d'une part avec le GM-CSF et, d'autre part, avec un muramyl-peptide et notamment un Murabutide ; en effet, comme nous l'avons vu, la cytokine a un effet thérapeutique chez l'homme de diminution des tumeurs ou d'une infection virale, mais parmi tous ces effets secondaire, elle confère au patient un neutropénie ou une leucopénie grandement préjudiciable.
         Le GM-CSF, quant à lui, lorsqu'il est administré seul confère des effets secondaires de type inflammatoires très importants aux patients. Mais, il a également un effet antineutropénique ou antileucopénique par l'augmentation du nombre de neutrophiles et de leucophiles dans le sang ; nous avons vu que le muramyl-peptide, tel le Murabutide ou le Muramétide, répare également l'effet neutropénique
         ou leucopénique des cytokines.
         L'association du GM-CSF, d'une cytokine, par exemple de l'interféron ou de l'IL-2, et du muramyl-peptide est donc une association de trois produits : le Murabutide et le GM-CSF ayant un effet réparateur cumulatif de la neutropénie et permettant d'éliminer les effets secondaires du GM-CSF seul.
   **C)** On observe également une production importante d'IL-1 RA et de STNF-R en l'absence de sécrétion de TNF, d'IL-1 et d'IL-8. Ceci conduit à un second volet d'applications. Tout d'abord vers la prévention et le traitement du choc septique dans lequel l'IL-1 et le TNF jouent un rôle primordial. Les autres indications sont toutes celles où il est necessaire de s'opposer à l'inflammation, comme dans les cas d'arthrites rhumatoïdes ou de certaines maladies auto-immunes ou de maladies induites dans le cas de transplantations de moelle, de tissus ou d'organes, par une réaction du greffon contre l'hôte.
**D)** Naturellement l'association est tout aussi utile pour le traitement de maladies virales telles les hépatites chroniques C ou B ou des infections virales respiratoires, qu'il s'agisse d'un traitement dans lequel la cytokine est directement impliquée en sa capacité anti-virale (par exemple cas de l'interféron) ou d'un traitement de substitution ou s'intercalant entre des traitements antiviraux qui doivent être interrompus par intermittences (par exemple inhibition de la multiplication virale de HIV par l'AZT ou d'autres dérivés de nucléosides ayant une spectre d'action antivirale similaire).

D'une façon générale, l'invention concerne donc toute association répondant aux critères sus-indiqués, contenant, le cas échéant, en outre un véhicule pharmaceutiquement acceptable. Sans qu'il y ait lieu de s'y limiter ces compositions pharmaceutiques sont avantageusement constituées de solutions pouvant être administrées par voie parentérale, notamment sous-cutanée, intraveineuse, ou par perfusion. Les muramyl-peptides peuvent être administrés oralement soit seuls, soit sous une forme galénique assurant leur protection (par exemple, en capsulation dans des liposomes capsules ou microsphères permettant le franchissement de la barrière gastrique. Il en est de même pour les cytokines utilisées, notamment les interférons.

Dans le cas de l'association interféron et muramyl-peptide, les doses préférées d'IFN pour une injection seront de l'ordre de 5 x 10⁴ à 5 x 10⁶ (0.05M à 5M) Unités internationales et les doses de muramyl-peptide, notamment de muramétide ou de murabutide, sont de l'ordre de 10 µg à 250 µg/Kg.

Pour le cas des associations GM-CSF et muramyl-peptides, des doses préférées seront respectivement de 1 à 25 µg/kg de GM-CSF pour 10 µg à 250 µg/Kg de muramyl-peptides.

Il va de soi que les doses indiquées ci-dessus n'ont d'autre valeur que celle d'illustrer l'invention. Il relève naturellement du clinicien de déterminer quelles seront les doses à associer des constituants de l'association, selon l'état du patient et de l'affection dont il souffre.

L'invention concerne aussi l'association avec l'IL-2 qui possède des activités anti-tumorales reconnues, en particulier vis-à-vis des métastases du carcinome rénal ou d'autres cancers, mais dont l'index thérapeutique est extrêmement faible. Les doses préférées pour une injection seraient de 1 à 10 millions d'unités Kg/jour d'IL-2 associées à de 10 µg à 250 µg/Kg de muramyl-peptides.

A titre d'exemples supplémentaires, l'invention concerne aussi les associations avec un muramyl-peptide du genre indiqué, de IL-6, de IFNγ et/ou du facteur TGFβ (abréviation anglaise de "Transforming Growth Factor" ou "facteur de transformation de la croissance"). On leur attribue une implication importante dans la régulation négative d'immunoglobulines de classe IgE, responsables de phénomènes allergiques. Par "régulation négative", il faut entendre une réduction de la production d'immunoglobulines de classe IgE relativement à la production d'autres immunoglobulines notamment des IgA et IgG. L'administration de l'association d'une ou plusieurs de ces cytokines en association avec un muramyl-peptide permet une modulation du profil des immunoglobulines synthétisées par les cellules du système immunitaire, dans un sens favorisant la production de IgG et IgA par rapport à celle des IgE, voire même au détriment de celles-ci. Ce résultat témoigne de la possibilité d'utiliser l'association conforme à l'invention pour le traitement des allergies en thérapeutique.

Les doses de chacun des constituants de l'association, notamment lorsqu'il s'agit de les administrer par voie parentérale, seraient notamment les suivantes, pour environ de 10 µg à 250 µg/Kg de muramyl-peptide :
- de 0,05 M à 10 M d'UI d'IFNγ/kg et par jour, de préférence 0,5 M d'UI/kg/jour à 5 M d'UI/kg/jour et encore de préférence de 1 M d'UI/kg/jour à 5 M d'UI/kg/jour,
- de 0,5 µg à 100 µg/Kg de TGFβ, et/ou
- de 0,1 µg à 25 µg/Kg de IL-6.

Il va enfin de soi que les revendications qui suivent ne peuvent qu'étendre leurs effets à des compositions, qui mettraient en jeu en association avec la cytokine choisie, une molécule du type muramyl-peptide utilisable en clinique humaine ou une molécule qui en présenterait les propriétés biologiques essentielles. On mentionnera à titre d'exemples les produits suivants :
- Nac Mur-L-Thr-D isoGln-sn glycéryl dipalmitoyle
- NAc-Mur-L-Ala-D-isoGln-L-Ala-2 (1',2'dipalmitoyl)-sn-glycéro-3'-phosphoryléthylamide (MTP-PE) ;
- N₂ (Nac-Mur-L-Ala-D-isoGln) N₆-stearoyl-L-Lysine (Muroctasine) ou MDP-Lys (L18) ;
- NAc-glucosaminyl-NAc-Mur-L-Ala-D-isoGln-L-Ala-Glyceryl-dipalmitate (DTP-GDP) ou NAc-glucosaminyl-NAc
- Mur-L-Thr-D-iso-Gln-L-Ala-Glycéryl-dipalmitate ;
- Nac-Mur-L-Thr-D-isoGln;
- Nac-Mur-D-Ala-D-isoGln-1,2-dipalmitoyl-sn-glycérol
- Nac-Mur-D-Thr-D-isoGln-1,2-dipalmitoyl-sn-glycérol
- Les analogues du MDP dans lesquels le 2^{ème} acide aminé, la glutamine est remplacée par une Norleucine, et qui sont dépourvus d'activité pyrogène ;
- Les dérivés ou analogues (obtenus par fractionnement ou mieux par synthèse) de produits bactériens suffisamment dépourvus de toxicité, notamment de pyrogénicité, parmi lesquels des endotoxines, notamment monomères ou peptidoglycanes, capables d'activer les cellules immunologiquement compétentes et d'induire des cytokines chez l'homme.

## Revendications

1. Composition thérapeutique qui comprend au moins une cytokine humaine, naturelle ou recombinante, sélectionnée dans le groupe comportant un interféron, un CSF et une interleukine, en association avec au moins un muramyl peptide hydrophile présentant la formule suivante: 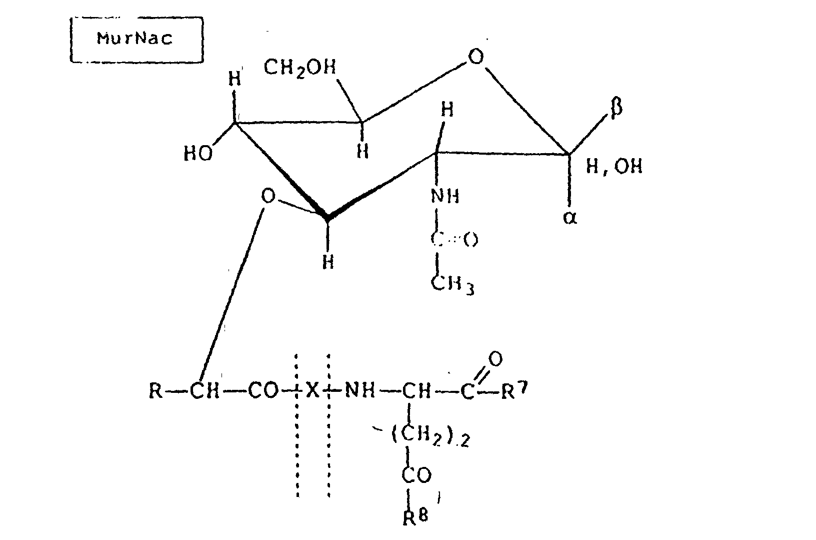 dans laquelle le groupe R est un groupe méthyle;
X représente L-alanyl ou L-thréonyl;
R⁷ est une groupe C(CH₂)_{X'}H ou X' = 1, 2, 3 ou 4 ; et
R⁸ est un groupe amino ou O(CH₂)_{X"}H ou X"=1, 2, 3 ou 4.

2. Composition selon la revendication 1, dans laquelle ledit muramyl peptide induit in vivo une augmentation de la synthèse de l'IL-6 ou du GM-CSF ou des deux.

3. Composition selon la revendication 1 ou la revendication 2 dans laquelle
R représente CH₃;
X représente L-alanyl ou L-thréonyl;
R⁷ est un group O(CH₂)_{X'} où X' = 1, 2, 3 ou 4; et
R⁸ est un groupe amino ou O(CH₂)_{X"} où X" = 1, 2, 3 ou 4.

4. Composition selon l'un des revendications 1 à 3, dans laquelle ledit muramyl peptide est sélectionné dans le groupe comportant le muramétide, le murabutide et leurs homologues et dans laquelle un résidu L-thréonyl est substitué au résidu L-alanyl du groupe muramyl peptide.

5. Composition selon l'une des revendications 1 à 4, dans laquelle ladite cytokine humaine est l'interféron-β.

6. Composition selon l'une des revendications 1 à 4, dans laquelle ladite cytokine humaine est l'interféron-α.

7. Composition selon l'une des revendications 1 à 4, dans laquelle ladite cytokine humaine est un CSF.

8. Composition selon la revendication 7, dans laquelle ledit CSF est GM-CSF.

9. Composition selon l'une des revendications 1 à 8, dans laquelle le muramyl peptide est choisi parmi ceux dans lesquels R⁷ est OC₄H₉ ou O-CH₃ et R⁸ est NH₂ ou O-CH₃.

10. Composition selon l'une des revendications 1 à 9, dans laquelle le muramyl peptide est celui dans lequel R⁷ est OC₄H₉ et R⁸ est NH₂.

11. Composition selon l'une des revendications 1 à 10, dans laquelle le muramyl peptide est celui dans lequel R⁷ est O-CH₃ et R⁸ est O-CH₃.

12. Utilisation d'une composition selon l'une des revendications 1 à 11, pour la fabrication d'un médicament destiné au traitement des cancers, des infections, des maladies ou déficiencies génétiques.

13. Utilisation d'une composition selon l'une des revendications 1 à 11 pour la fabrication d'un médicament destiné à l'induction du système granulocytaire chez un patient.

14. Utilisation d'une composition selon l'une des revendications 1 à 11 pour la fabrication d'un médicament destiné à promouvoir une reconstitution rapide du système hématpoïetique chez un patient dont le système immunitaire a été inhibé.

15. Utilisation d'une composition selon l'une des revendications 1 à 11 pour la fabrication d'un médicament destiné au traitement de maladies virales.

16. Utilisation d'une composition selon l'une des revendications 1 à 11 pour la fabrication d'un médicament destiné à la prévention ou au traitement du choc septique.

17. Utilisation d'une composition selon l'une des revendications 1 à 11 pour la fabrication d'un médicament destiné au traitement ou à la prévention des processus inflammatoires.

18. Utilisation selon l'une quelconque des revendications 12 à 17 dans laquelle la cytokine est GM-CSF ou IL-2.

19. Utilisation selon l'une quelconque des revendications 12 à 18 pour la production d'un médicament permettant l'administration de 10 à 350 µg/kg/jour de muramyl peptide.

20. Utilisation selon l'une quelconque des revendications 12 à 18 pour la production d'un médicament permettant l'administration de 50 à 200 µg/kg/jour de muramyl peptide.

21. Utilisation selon l'une quelconque des revendications 12 à 17 pour la production d'un médicament permettant l'administration de 0,05 à 5 µg/kg/jour de cytokine qui est l'interféron.

22. Utilisation selon l'une quelconque des revendications 12 à 17 pour la production d'un médicament permettant l'administration de 3 à 10 MU/kg/jour de cytokine qui est IL-2.

23. Utilisation selon l'une quelconque des revendications 12 a 17 pour la production d'un médicament permettant l'administration de 5 à 10 µg/kg/jour de cytokine qui est GM-CSF.

## Claims

1. A therapeutic composition comprising at least one natural or recombinant human cytokine selected from the group formed by an interferon, a CSF and an interleukin, in association with at least one hydrophilic muramyl peptide with the following formula: 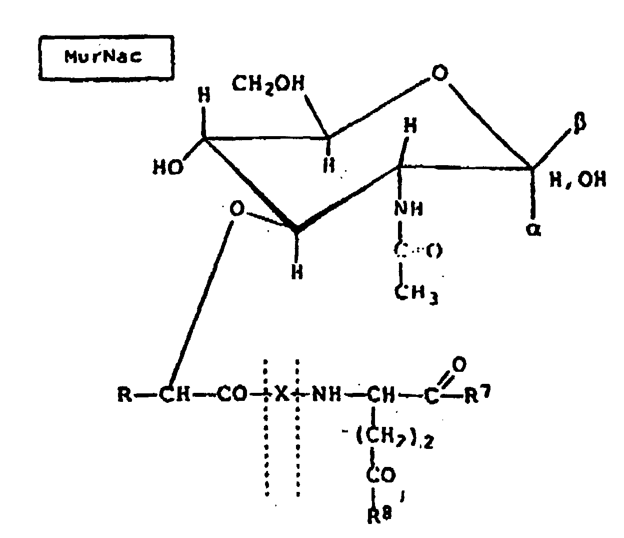 in which:
group R is a methyl group;
X represents L-alanyl or L-threonyl;
R⁷ is a O(CH₂)_{X'}H group in which X' = 1, 2, 3 or 4; and
R⁸ is an amino group or an O(CH₂)_{X"}H group in which X" = 1, 2, 3 or 4.

2. A composition according to claim 1, in which said muramyl peptide induces an increase in the synthesis of IL-6 or GM-CSF or both in vivo.

3. A composition according to claim 1 or claim 2, in which:
R represents CH₃;
X represents L-alanyl or L-threonyl;
R⁷ is a O(CH₂)_{x'} group in which X' = 1, 2, 3 or 4; and
R⁸ is an amino group or an O(CH₂)_{X"} group in which X" = 1, 2, 3 or 4.

4. A composition according to one of claims 1 to 3, in which said muramyl peptide is selected from the group formed by murametide, murabutide and their homologues and in which a L-threonyl residue is substituted for the L-alanyl residue of the muramyl peptide group.

5. A composition according to one of claims 1 to 4, in which said human cytokine is interferon-β.

6. A composition according to one of claims 1 to 4, in which said human cytokine is interferon-α.

7. A composition according to one of claims 1 to 4, in which said human cytokine is a CSF.

8. A composition according to claim 7, in which said CSF is GM-CSF.

9. A composition according to one of claims 1 to 8, in which the muramyl peptide is selected from those in which R⁷ is OC₄H₉ or O-CH₃ and R⁸ is NH₂ or O-CH₃.

10. A composition according to one of claims 1 to 9, in which the muramyl peptide is that in which R⁷ is OC₄R₉ and R⁸ is NH₂.

11. A composition according to one of claims 1 to 10, in which the muramyl peptide is that in which R⁷ is O-CH₃ and R⁸ is O-CH₃.

12. Use of a composition according to one of claims 1 to 11, for the production of a drug for the treatment of cancers, infections, diseases or genetic deficiencies.

13. Use of a composition according to one of claims 1 to 11, for the production of a drug for inducing the granulocyte system in a patient.

14. Use of a composition according to one of claims 1 to 11, for the production of a drug for promoting rapid reconstitution of the haematopoietic system in a patient in whom the immune system has been inhibited.

15. Use of a composition according to one of claims 1 to 11, for the production of a drug for the treatment of viral diseases.

16. Use of a composition according to one of claims 1 to 11, for the production of a drug for preventing or treating septic shock.

17. Use of a composition according to one of claims 1 to 11, for the production of a drug for the treatment or prevention of inflammatory processes.

18. Use according to one of claims 12 to 17, in which the cytokine is GM-CSF or IL-2.

19. Use according to one of claims 12 to 18 for the production of a drug for administering 10 to 350 µg/kg/day of muramyl peptide.

20. Use according to one of claims 12 to 18 for the production of a drug for administering 50 to 100 µg/kg/day of muramyl peptide.

21. Use according to one of claims 12 to 17 for the production of a drug for administering 0.05 to 5 µg/kg/day of a cytokine, namely interferon.

22. Use according to one of claims 12 to 17 for the production of a drug for administering 3 to 10 MU/kg/day of a cytokine, namely IL-2.

23. Use according to one of claims 12 to 17 for the production of a drug for administering 5 to 10 µg/kg/day of a cytokine, namely GM-CSF.

## Patentansprüche

1. Therapeutische Zusammensetzung umfassend mindestens ein natürliches oder rekombinantes menschliches Cytokin, ausgewählt aus der Gruppe bestehend aus einem Interferon, einem CSF und einem Interleukin, in Verbindung mit mindestens einem hydrophilen Muramylpeptid, welches folgende Formel aufweist: 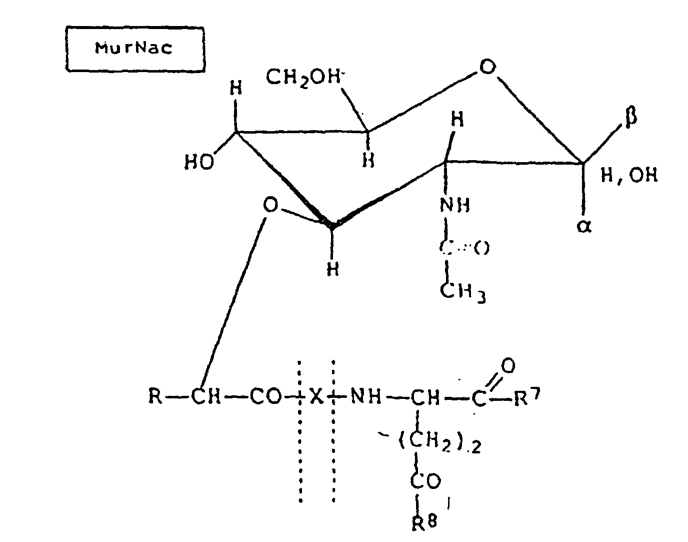 in welcher R eine Methylgruppe ist;
X eine L-Alanyl- oder L-Threonylgruppe darstellt;
R⁷ ein Rest O(CH₂)_{x'}H ist, wobei X'=1, 2, 3 oder 4, und
R⁸ eine Aminogruppe oder ein Rest O(CH₂)_{x"}H ist, wobei X"=1, 2, 3 oder 4.

2. Zusammensetzung nach Anspruch 1, wobei das Muramylpeptid in vivo eine Steigerung der Synthese von IL-6 oder von GM-CSF oder von beiden verursacht.

3. Zusammensetzung nach Anspruch 1 oder 2, wobei
R eine Methylgruppe ist;
X eine L-Alanyl- oder L-Threonylgruppe ist;
R⁷ ein O(CH₂)_{X'}-Rest ist, wobei X' = 1, 2, 3 oder 4; und
R⁸ eine Aminogruppe oder ein Rest O(CH₂)_{X"} ist, wobei X" =1, 2, 3 oder 4.

4. Zusammensetzung nach einem der Ansprüche 1 bis 3, wobei das Muramylpeptid ausgewählt ist aus der Gruppe bestehend aus dem Murametid, dem Murabutid und seinen Homologen und wobei ein L-Threonylrest durch einen L-Alanylrest der Muramylpeptidgruppe ersetzt ist.

5. Zusammensetzung nach einem der Ansprüche 1 bis 4, wobei das menschliche Cytokin Interferon-β ist.

6. Zusammensetzung nach einem der Ansprüche 1 bis 4, wobei das menschliche Cytokin Interferon-α ist.

7. Zusammensetzung nach einem der Ansprüche 1 bis 4, wobei das menschliche Cytokin ein CSF ist.

8. Zusammensetzung nach Anspruch 7, wobei CSF GM-CSF ist.

9. Zusammensetzung nach einem der Ansprüche 1 bis 8, wobei das Muramylpeptid ausgewählt ist aus denjenigen, in denen der Rest R⁷ OC₄H₉ oder O-CH₃ ist, und denjenigen, in denen der Rest R⁸ NH₂ oder O-CH₃ ist.

10. Zusammensetzung nach einem der Ansprüche 1 bis 9, wobei das Muramylpeptid dasjenige ist, in dem der Rest R⁷ OC₄H₉ und der Rest R⁸ NH₂ ist.

11. Zusammensetzung nach einem der Ansprüche 1 bis 10, wobei das Muramylpeptid dasjenige ist, in dem der Rest R⁷ O-CH₃ und der Rest R⁸ O-CH₃ ist.

12. Verwendung einer Zusammensetzung nach einem der Ansprüche 1 bis 11, zur Herstellung eines Medikaments zur Behandlung von Krebs, Infektionen, Krankheiten oder genetischen Defekten.

13. Verwendung einer Zusammensetzung nach einem der Ansprüche 1 bis 11 zur Herstellung eines Medikaments zur Induktion des granulocytären Systems bei einem Patienten.

14. Verwendung einer Zusammensetzung nach einem der Ansprüche 1 bis 11 zur Herstellung eines Medikaments zur schnellen Wiederherstellung des hämatopoetischen Systems bei einem Patienten mit beeinträchtigtem Immunsystem.

15. Verwendung einer Zusammensetzung nach einem der Ansprüche 1 bis 11 zur Herstellung eines Medikaments zur Behandlung von viralen Erkrankungen.

16. Verwendung einer Zusammensetzung nach einem der Ansprüche 1 bis 11 zur Herstellung eines Medikaments zur Vorbeugung oder zur Behandlung des septischen Schocks.

17. Verwendung einer Zusammensetzung nach einem der Ansprüche 1 bis 11 zur Herstellung eines Medikaments zur Behandlung oder zur Vorbeugung von entzündlichen Prozessen.

18. Verwendung nach einem der Ansprüche 12 bis 17, wobei das Cytokin GM-CSF oder IL-2 ist.

19. Verwendung nach einem der Ansprüche 12 bis 18 zur Herstellung eines Medikaments, welches die Verabreichung von 10 bis 350 µg/kg/Tag Muramylpeptid erlaubt.

20. Verwendung nach einem der Ansprüche 12 bis 18 zur Herstellung eines Medikaments, welches die Verabreichung von 50 bis 200 µg/kg/Tag Muramylpeptid erlaubt.

21. Verwendung nach einem der Ansprüche 12 bis 17 zur Herstellung eines Medikaments, welches die Verabreichung von 0,05 bis 5 µg/kg/Tag des Cytokins Interferon erlaubt.

22. Verwendung nach einem der Ansprüche 12 bis 17 zur Herstellung eines Medikaments, welches die Verabreichung von 3 bis 10 MU/kg/Tag des Cytokins IL-2 erlaubt.

23. Verwendung nach einem der Ansprüche 12 bis 17 zur Herstellung eines Medikaments, welches die Verabreichung von 5 bis 10 µg/kg/Tag des Cytokins GM-CSF erlaubt.
